# EUROPEAN PATENT SPECIFICATION

(11) **EP 2 477 670 B1**
(45) Date of publication and mention of the grant of the patent: **23.11.2016**
(21) Application number: 10745117.1
(22) Date of filing: 16.08.2010
(51) Int. Cl.: A61L 29/14, A61L 29/16, A61L 31/14, A61L 31/16, A01N 25/02, A01N 31/02, A01N 31/16, A01N 47/44

(54) **ANTI-INFECTIVE LUBRICANT FOR MEDICAL DEVICES AND METHODS FOR PREPARING THE SAME**
ANTIINFEKTIVES SCHMIERMITTEL FÜR MEDIZINPRODUKTE VERFAHREN ZU IHRER HERSTELLUNG
LUBRIFIANT ANTI-INFECTIEUX POUR DISPOSITIFS MÉDICAUX ET SES PROCÉDÉS DE PRÉPARATION

(30) Priority: 17.09.2009 US 561863
(43) Date of publication of application: 25.07.2012
(73) Proprietor: Becton Dickinson and Company, Franklin Lakes, NJ 07417-1880 (US)
(72) Inventor: HOANG, Minh Quang, Sandy, Utah 84093 (US); KHAN, Mohammad A., Sandy, Utah 84092 (US)
(74) Representative: dompatent von Kreisler Selting Werner - Partnerschaft von Patent- und Rechtsanwälten mbB
(86) International application number: PCT/US2010/045616
(87) International publication number: WO 2011/034675

(56) References cited:
- WO-A1-2008/031601
- WO-A2-2011/005951
- US-A- 6 120 784
- US-A1- 2005 048 124

## Description

### BACKGROUND OF THE INVENTION

This disclosure relates generally to lubricious antiseptic coating materials. In particular, this disclosure discusses a silicone oil-based antiseptic coating material containing various solvents to achieve mutual miscibility of the material components.

In the fields of medicine and health care, a patient's skin may be punctured in a variety of manners and for a variety of reasons. In one example, a patient's skin is cut with a sharp object, such as a scalpel, for surgical reasons. In another example, a cannula or an intravenous ("IV") catheter is forced through the patient's skin into an interior space, such as the patient's vasculature. In this example, the cannula or IV catheter can be used for infusing fluid (e.g., saline solution, medicaments, and/or total parenteral nutrition) into the patient, withdrawing fluids (e.g., blood) from the patient, and/or monitoring various parameters of the patient's vascular system.

However, when a patient's skin is punctured, the likelihood of infection in the patient increases. Indeed, it is estimated that each year hundreds of thousands of patients in the United States alone develop some form of bloodstream infection that is caused by pathogens that were communicated to the patient through or because of an IV catheter or another IV access device, such as a hypodermic needle. Many of the bacterial pathogens that cause these catheter-related bloodstream infections are common skin colonizers, or flora that exist on the patient's skin, and are often believed to enter the patient's body through the catheter insertion site.

Often, these catheter-related bloodstream infections cause patient illness and, in some cases, death. Furthermore, because some infections are caused by bacterial strains (e.g., Methicillin-resistant *Staphylococcus aureus* ("MRSA") and Vancomycin-resistant *Enterococci* ("VRE")) that are resistant to antibiotics, such infections can be hard to treat and may be increasing in prevalence. Additionally, because patients that have a bloodstream infection may require additional medical treatment, catheter-related bloodstream infections may also be associated with increased medical costs.

In an attempt to limit bloodstream infections (i.e., catheter-related infections) in hospital, outpatient, home care, and other health care settings, many have attempted to apply various antiseptic coatings to medical devices and equipment. The antiseptic coating is provided as a barrier to prevent the growth or colonization of pathogens commonly associated with bloodstream infections. However, such antiseptic coatings are not without their shortcomings. For example, many of the antipathogenic agents within the coating materials are water soluble and therefore are easily washed away from the medical devices during contact with fluids associated with a patient. Furthermore, some of the most effective antipathogenic agents leave a sticky or tacky residue when dried thereby making it difficult to work with the medical device for procedures requiring a lubricious interface between the medical device and the patient.

WO 2008/031601) discloses a formulation for preparing an antimicrobial lubricious hydrophilic coating on medical devices like catheters, which formulation comprises a hydrophilic polymer, particles of metallic silver and a carrier liquid.

Thus, while techniques currently exist that are used to coat or otherwise treat the surfaces of medical devices to prevent infection, challenges still exist. Accordingly, it would be an improvement in the art to augment or even replace current techniques with other techniques.

### BRIEF SUMMARY OF THE INVENTION

The present application relates to a lubricious, antiseptic coating material capable of being applied to an intravascular device to kill or prevent the growth of a wide range of pathogens. The antiseptic coating material includes one or more antipathogenic agents selected to kill or inhibit the growth of various pathogens. The antipathogenic agent is soluble in at least one of water and a lower alcohol having no more than two carbon atoms. Accordingly, the coating material further includes a solvent for dissolving the antipathogenic agent within the coating material.

Further components of the antiseptic coating material include a lubricious agent to ensure a lubricious interface between the coated medical device and the patient. The lubricious agent is selected from one or more silicone oils. Silicone oils are generally soluble in at least one of a hydrocarbon, a ketone, a halogenated hydrocarbon, and higher alcohols having at least 3 carbon atoms. Accordingly, the coating material further includes a solvent for dissolving the lubricious agent within the coating material.

An important feature of the present invention is the mutual miscibility of the various components of the antiseptic coating material. Thus, in the present invention various combinations of mutually miscible antipathogenic agents, antipathogenic solvents, lubricious agents and lubricious agent solvents are provided to ensure a homogenous coating material. In some embodiments, the coating material further includes a polyethoxylated surfactant to further ensure miscibility of coating's various components. In other embodiments, pluralities of solvents are combined to achieve mutually miscible and homogenous coating material.

In some embodiments, the antiseptic coating material is modified to have any suitable characteristic desired to enable application of the material on a desired surface. For example, in some embodiments the coating material is provided in a liquid form that is applied to the surface of a medical device by dipping or brushing. In other embodiments, the coating material is provided in at least one of a gel, a cream, a foam, an aerosol, or another fluid having a desired consistency and viscosity.

The coating material of the present invention may be applied to any desired surface. For example, in some embodiments the coating material is applied directly to at least one of the outer and inner surfaces of an intravascular medical device. In other embodiments, the coating material is applied directly to the skin of patient prior to puncturing or otherwise compromising the skin for a medical procedure.

### DETAILED DESCRIPTION OF THE INVENTION

In order to provide a thorough understanding of the invention, the following description discusses specific details. The skilled artisan, however, would understand that the invention can be practiced without employing these specific details. Indeed, the invention can be modified in any suitable manner and can be used in conjunction with any suitable chemical, apparatus, and technique conventionally used in the industry. Thus, the following more detailed description of the embodiments of the invention is not intended to be limiting in scope, but is merely representative of some presently preferred embodiments. Additionally, while the following discussion focuses on using the invention in health care settings, the antiseptic material may be used in any suitable setting.

Generally, the present invention relates to a lubricious, antiseptic coating material capable of being applied to an intravascular device to kill or prevent the growth of a wide range of pathogens. As used herein, the terms pathogen and pathogens may include any potentially infectious microorganism, such as bacteria (e.g., undulating bacteria, gram-negative bacteria, gram-positive bacteria, aerobic bacteria, anaerobic bacteria, mycobacteria, spriochetes, *Staphylococcus epidermis, Staphylococcus aureus, Escerchia coli, Proteus vulgaris, Streptococcus faecalis, Klebsiella, Ertterobacter aerogenes*, *Proteus mirabilis,* and the like), fungi (e.g., fungal spores, *Aspergillus niger, Aspergillus flavus, Rhizopus nigricans, Cladosporium herbarium, Epidermoplyton Floccosum, Trichoplyton mentagroplytes, Histoplasma capsulatum,* and the like), yeast (e.g., *Saccharomyces cerevisiae, Candida albicans,* and the like), virus or other potentially hazardous microbes.

A preferred application of the present invention is to apply the antiseptic coating material directly to the outer surfaces of an intravascular device, such as an intravenous catheter. In some embodiments, the coating material is applied only to those surfaces of the intravascular device that directly contact the patient. In other embodiments, the coating material is applied to any surface of the intravascular device susceptible to pathogen colonization. Thus, when the coated intravascular device is placed through the skin of the patient, the antiseptic properties of the coating material kill or prevent the growth of pathogens that may potentially lead to catheter-related bloodstream infection (CRBSI).

The antiseptic coating material includes various mutually miscible components selected to provide a coating material that is both anti-infective and lubricious. Generally, all catheters in use for the care of patients are lubricated with various viscosities of silicone oils. Silicone oils are silicon analogues of carbon based organic compounds that form relatively long and complex molecules based on silicon rather than carbon. Silicone oil chains are formed of alternating siloxane atoms that are substituted with various other species at the tetravalent silicon atoms.

Embodiments of the present invention contain silicone oils which serve as lubricating agents within the antiseptic material. Silicone oils in accordance with embodiments of the present invention are selected for their lubricious properties and their miscibility within the antiseptic coating material. Furthermore, the silicone oils are selected for their general lack of solubility in water and other fluid associated with a patient, such as blood, sweat, water and urine. Once applied to the surface of an intravascular device, the silicone oil component prevents the coating material from being easily removed from the surface of the device. Thus, the silicone oil preserves the antiseptic properties of the coating by ensuring adhesion between the coating material and the extravascular device. Non-limiting examples of silicone oils used in accordance with the present invention include dimethicone, trifluoropropylmethylsiloxane, and combinations thereof. One of skill in the art will appreciate that other silicone oils may be successfully used in accordance with the teachings of the present invention.

An important aspect of the present invention requires that each component of the antiseptic coating material be mutually miscible so as to provide a generally homogenous coating material. Accordingly, the coating material comprises various miscibly compatible components, including compatible solvents to dissolve the various components into mutually miscible solutions that are mixed to provide the coating material. Thus, while some components may be soluble in silicone oils, other components may be insoluble and therefore require an additional solvent. Additionally, in some embodiments a solvent is required to enable the silicone oil to dissolve within the coating material.

For example, in some embodiments the antiseptic coating material further includes a solvent capable of miscibly dissolving the silicone oil. A desirable silicone oil solvent will generally be compatible or miscible with the other components of the coating material. Suitable solvents may include various hydrocarbons, ketones, halogenated hydrocarbons, and alcohols having at least 3 carbon atoms. Non-limiting examples of silicone oil solvents compatible with and in accordance with the present invention include methyl nonafluorobutyl ether, ethyl nonafluorobutyl ether, and trans-1,2-dichloroethylene, and polyethoxylated surfactants. In some embodiments, the coating material includes a single silicone oil solvent. In other embodiments, the coating material includes a mixture of silicone oil solvents.

An important component of the antiseptic coating material is an antipathogenic agent selected to kill or prevent growth of various pathogens. A desirable antipathogenic agent is generally selected based on its ability to inhibit pathogenic activity, and to miscibility combine with the other components of the coating material. Non-limiting examples of compatible, highly effective antipathogenic agents include chlorhexidine diacetate, chlorhexidine gluconate, triclosan, benzalkonium chloride, para-chloro-meta-xylenol (PCMX), and other agents known to inhibit bacterial growth.

Most highly effective antipathogenic agents are soluble in water or lower alcohols, such as alcohols having no more than two carbon atoms. However, these agents are generally not soluble in silicone oils, thereby making it difficult to dissolve some antipathogenic agents, such as chlorhexidine diacetate and triclosan, in silicone oils. Therefore, in some embodiments of the present invention it is desirable to include within the coating material a solvent capable of miscibly dissolving the antipathogenic agent. Non-limiting examples of compatible antipathogenic agent solvents include water and lower alcohols, such as methanol and ethanol.

The antiseptic coating material may include any suitable concentrations of the previously mentioned components necessary to provide a mutually miscible, anti-infective coating capable of being applied to an intravascular device. Referring now to Table 1, nine formulations of various antiseptic coating materials are shown, in accordance with representative embodiments of the present invention.

**Table 1**

| | Formulation Numbers (% w/w) | | | | | | | | |
|---|---|---|---|---|---|---|---|---|---|
| Ingredients | 1 | 2 | 3 | 4 | 5 | 6 | 7 | 8 | 9 |
| Methyl Nonafluorobutyl ether | 43.35 | 29.75 | 2.64 | 8.1 | 45.8 | 7.3 | -- | -- | -- |
| Ethyl Nonafluorobutyl ether | -- | -- | -- | 16.2 | | 14.6 | -- | -- | -- |
| Trans-1,2-dichloroethylene | 43.35 | 29.75 | 2.23 | 56.7 | 38.76 | 51.10 | -- | -- | -- |
| Ethanol, USP | 10.00 | 30.00 | 5.13 | 15.0 | 12.34 | 20.0 | 10.0 | 13.93 | 13.85 |
| 12500 cst Silicone | 3.00 | 3.00 | -- | 3.00 | 3.00 | 3.00 | 3.00 | -- | -- |
| Dimethylsiloxane and Trifluoropropylmethylsiloxane | -- | -- | 89.5 | -- | -- | -- | -- | 85.58 | 84.08 |
| Hydrochlorofluorocarbon | -- | -- | -- | -- | -- | -- | 89.8 | -- | -- |
| Cremophor EL ^{®} | -- | -- | -- | -- | -- | -- | -- | 0.99 | 1.09 |
| Triclosan | -- | -- | -- | -- | -- | -- | -- | -- | 0.49 |
| Chlorhexidine diacetate | 0.30 | 7.50 | 0.50 | 1.00 | 0.1 | 4.00 | 0.20 | 0.50 | 0.49 |

In some embodiments, the silicone oil added to the coating material is highly concentrated thereby requiring the addition of a relatively small amount. For example, formulations 1, 2 and 4-6, each comprise highly viscous and concentrated 12,500 cSt silicone thereby resulting in only 3% of the total weight of the coating material being attributed to the silicone oil. Conversely, formulations 3, 8 and 9 each contain a lower concentrated mixture of dimethylsiloxane and Trifluoropropylmethylsiloxane silicone oils thereby resulting in approximately 84% to 90% of total weight of the formulation being attributed to the silicon oils. Finally, formulation 7 comprises both the highly concentrated 12,500 cSt silicone (3%) and the mixture of silicone oils (89.8%) for a total percent weight of approximately 93% being attributed to the silicone oils.

Each formulation includes at least one solvent to aid in solubilising the silicone oil. In some embodiments, multiple silicone oil solvents are added to the formulation. For example, formulation 7 includes a single halogenated hydrocarbon solvent, while formulations 1-3 and 5 each contain two halogenated hydrocarbon solvents, each solvent or combinations of solvents being provided to dissolve their respective silicone oils. Similarly, formulations 4 and 6 each contain three halogenated hydrocarbon solvents. In contrast, formulations 8 and 9 do not contain any halogenated hydrocarbon solvents, but rather contain a polyethoxylated surfactant, Cremophor EL ®. For formulations containing non-halogenated solvents, such as formulations 8 and 9, the addition of a small, polyethoxylated surfactant was found to improve the miscibility of the silicone oils within the coating material. Therefore, in some embodiments of the present invention, the silicone oil solvent must include at least one of a halogenated hydrocarbon solvent and a polyethoxylated surfactant.

Each formulation includes at least one antipathogenic agent. An antipathogenic agent in accordance with the present invention may include any single compound, chemical, reagent, medication, substrate or solution capable of killing or otherwise hindering the growth of a pathogen. Furthermore, an antipathogenic agent in accordance with the present invention may include any combination of compounds, chemicals, reagents, medications, substrates or solutions capable of killing or otherwise hindering the growth of a pathogen. Common antipathogenic agents in accordance with various embodiments of the present invention include chlorhexidine diacetate, chlorhexidine gluconate, triclosan, benzalkonium chloride, para-chloro-meta-xylenol (PCMX), and other agents known to inhibit bacterial growth.

In some embodiments, an antipathogenic agent is selected based upon the agent's ability to inhibit a specific pathogen or class of pathogens. For example, formulations 1 through 8 each contain chlorhexidine diacetate in various concentrations from approximately 0.1% to 7.5% of the total weight of the coating material. Chlorhexidine diacetate is a chemical antiseptic that kills both gram-positive and gram-negative microbes. Chlorhexidine diacetate is also commonly used for its bacteriostatic properties. Therefore, formulations 1 through 8 are generally provided for those applications where prevention of bacterial growth is desired. In contrast, formulation 9 includes both chlorhexidine diacetate and triclosan antipathogenic agents. Triclosan is a potent wide spectrum antibacterial and antifungal agent. Thus, in addition to killing bacterial microbes, formulation 9 is also effective against fungal microbes, thereby making formulation 9 a broader antiseptic coating material.

As mentioned above, some embodiments of the present invention further include a solvent capable of miscibly dissolving the antipathogenic agent. Generally, this antipathogenic solvent is selected to dissolve the antipathogenic agent such that all of the components within the antiseptic coating material are mutually miscible. The antipathogenic solvent is selected from at least one of water and a lower alcohol containing no more than two carbon atoms. The formulations of Table 1 each contain the solvent ethanol from approximately 5.13% to 30.0% of the total weight of the coating material. The various concentrations of the ethanol solvent were selected based upon the concentrations of the other components as required to achieve mutual miscibility.

In some embodiments, the antiseptic coating material comprises more than one type of alcohol. In such embodiments, the coating material may comprise any suitable number of alcohols, including 2, 3, 4, or more alcohols. Additionally, the antipathogenic solvent may comprise any suitable combination of alcohols. In one example, the solvent comprises methanol and ethanol.

One having skill in the art will appreciate that mutual miscibility of the antiseptic coating material components may be achieved through the use of various solvents and combinations of solvents. One of skill in the art will also appreciate that some solvents may be included to intentionally increase or decrease the effectiveness of the coating material. For example, in some embodiments a selected solvent, such as an alcohol, may be included at a higher concentration to further inhibit the growth of a pathogen. In other embodiments, a selected solvent, such as an alcohol, may be combined with an emollient or moisturizer to counteract skin irritation and dryness associated with the solvent.

As mentioned above, in some embodiments the antipathogenic agent solvent comprises water. In such embodiments, the water may be provided to the antiseptic coating material in any suitable aqueous solution, including a dilute alcohol or other solution containing water. Nevertheless, in some embodiments, the water comprises purified water, such as United States Pharacopeia ("USP") water or de-ionized water. For example, where the antipathogenic solvent comprises water, the coating material may comprise any suitable amount of water. Indeed, in some embodiments, in addition to the silicone oil, the silicone oil solvent, the antipathogenic agent, alcohol, and/or any other suitable ingredient, the remaining portion of the coating material comprises water. In some embodiments, the coating material comprises from about 1% to about 99% water.

In some embodiments, the antiseptic coating further comprises at least one additional biocidal agent. In such embodiments, the additional biocidal agent may comprise any suitable chemical or chemicals that kill, reduce, or otherwise impede pathogen proliferation while allowing the antiseptic coating material to sanitize and lubricate the surfaces of the intravascular device, and be suitable for use on human skin. Some examples of suitable biocidal agents include silver and/or copper ions and nanoparticles (e.g., tinosan silver dihydrogen citrate), silver sulphadiazine, an imidozole, a triazole, an allyamine, phenol, hexachlorophene, an antibiotic, and a sulfonamide.

Where the antiseptic coating material comprises an additional biocidal agent, the coating material may comprise any suitable portion of the biocidal agent. In one example, an additional biocidal agent comprises from about 0.01% to about 10% of the total weight of the coating material. In another example, the biocidal agent comprises from about 0.1% to about 5% of the coating material, by weight. In still another example, the additional biocidal agent comprises from about 0.5% to about 2% of the antiseptic coating material, by weight.

In addition to the aforementioned ingredients, the antiseptic coating material may include any suitable ingredient, at any suitable concentration, which allows the coating material to lubriciously sanitize surfaces, be suitable for dermal use, and prevent colonization of pathogens. Some examples of such optional ingredients may include thickening agents, neutralizing agents, pH adjusters, metallic salts, dyes, fragrances, and/or other suitable chemicals.

The antiseptic coating material can also be modified to have any suitable characteristic desired to enable application of the material on a desired surface. For example, in some embodiments the coating material comprises a liquid that is applied to the surface of an intravenous device by dipping or brushing. In other embodiments, the coating material comprises at least one of a gel, a cream, a foam, an aerosol, or another fluid having a desired consistency/viscosity.

The antiseptic coating material may be used in any suitable manner. For example, the coating material may be disposed on and/or in a receptacle, from which the material may be dispensed or otherwise used to clean an object. In such instances, the coating material may be disposed on and/or in any suitable receptacle with any component, device or characteristic that allows it to be used with the coating material while allowing the coating material to act as intended. Some examples of suitable receptacles may include an absorbent material (e.g., a towelette, gauze, a swab, a swabstick, a sponge, a sponge with a feeding-fluid reservoir applicator, a fabric, a wad of fibers, etc.), a spray bottle, an aerosol dispenser, or any other suitable container.

Where the antiseptic coating material is disposed on and/or in an absorbent material, the material may comprise any suitable substance that is capable of absorbing the coating material, releasing some of the material when the absorbent material contacts (e.g., wipes) a surface, and which is suitable for use on human skin. Some examples of suitable substances may comprise cotton, paper, cellulose, wool, polyester, polypropylene, fabric, or another material that is capable of forming an absorbent object capable of applying the coating to a surface.

The coating material may be applied to virtually any surface. In one example, the coating material is applied directly to skin (e.g., to sanitize hands, to clean a portion of a patient's skin before the skin is punctured, to clean and care for a patient's skin after it is punctured, etc.). In another example, the coating material is applied to non-living objects, such as medical instruments, floors, chairs, door handles, tables, computer keyboards, computer mice, etc.

The coating material may be used to coat a surface in any suitable manner. For example, an object, such as a medical instrument (e.g., a catheter, a syringe, scalpel, or another object used in health care settings) can be coated with the antiseptic material. In this example, the coating material is applied to the object in any suitable manner, including by wiping (e.g., via an absorbent material), spraying, soaking, misting, immersing, or otherwise applying the coating material to the object. Additionally, in this example, when an object is coated with the antiseptic material, the coating material provides a lubricious layer of antipathogenic agent that is not easily removed through contact with a patient. Thus, the layer of antipathogenic material may remain on the object for some period of time and, thereby, act to prevent growth of pathogens and to reduce the amount pathogens that will colonize the object's coated surface.

### EXAMPLES

Antimicrobial efficacy of formulation 3 *(see* Table 1, above) was tested by zone of inhibition experiments, as follows. An antiseptic coating material was provided by miscibly combining chlorhexidine diacetate (0.49%), triclosan (0.49%), USP ethanol (13.85%), dimethylsiloxane and trifluoropropylmethylsiloxane (84.09%), and polyethoxylated surfactant Cremophor EL® (1.09%). The antiseptic coating material was then applied to the exterior surface of Becton Dickenson^{®} Q-Syte™ catheter components. A first set of coated catheter components were then rinsed in USP water to remove any unbound coating material, and a second set of coated catheter components were not rinsed. Finally, a third set of uncoated catheter components (control group) were rinsed separately in USP water.

Triplicate samples of *P. aeruginosa, S. aureus, E. coli,* and *C*. *albicans* pathogens were plated on agarose growth media. One of each sample received a rinsed coated catheter component, one of each sample received a non-rinsed coated catheter component, and one of each sample received an uncoated, control catheter component. The twelve samples were then incubated at 37°C for seven days. Each day, the samples were removed from the incubator and the zone of inhibition was measured. The results of the experiment are shown in Tables 2 through 4, below.

**Table 2**

| Fully Coated Catheter Component with Formulation 9 | | | | |
|---|---|---|---|---|
| Pathogen | *P. aeruginosa* | *S. aureus* | *E. coli* | *C. albicans* |
| day 1 | 2mm | 19mm | 9mm | 3mm |
| day 2 | 1mm | 18mm | 9mm | 5mm |
| day 3 | 0mm | 18mm | 11mm | NA |
| day 6 | 0mm | 16mm | 10mm | 5mm |
| day 7 | 0mm | 16mm | 10mm | 5mm |

**Table 3**

| Rinsed Coated Catheter Component with Formulation 9 | | | | |
|---|---|---|---|---|
| Pathogen | *P. aeruginosa* | *S. aureus* | *E. coli* | *C. albicans* |
| day 1 | 0mm | 21mm | 11mm | 1mm |
| day 2 | 0mm | 21mm | 11mm | 1mm |
| day 3 | 0mm | 20mm | 11mm | NA |
| day 6 | 0mm | 18mm | 9mm | 1mm |
| day 7 | 0mm | 18mm | 9mm | 1mm |

**Table 4**

| Rinsed Uncoated Catheter Component (control) | | | | |
|---|---|---|---|---|
| Pathogen | *P. aeruginosa* | *S. aureus* | *E. coli* | *C. albicans* |
| day 1 | 0mm | 0mm | 0mm | 0mm |
| day 2 | 0mm | 0mm | 0mm | 0mm |
| day 3 | 0mm | 0mm | 0mm | NA |
| day 6 | 0mm | 0mm | 0mm | 0mm |
| day 7 | 0mm | 0mm | 0mm | 0mm |

Table 2 shows the results from the pathogen samples containing the fully coated and non-rinsed catheter components. As shown, the antiseptic coating material of formulation 9 demonstrated significant inhibition of the *S. aureus* and *E. coli* pathogens over the seven day period. Furthermore, while C. *albicans* demonstrated slightly less inhibition than *S. aureus* and *E. coli, P. aeruginosa* showed significant resistance to formulation 9.

Table 3 shows the results from the pathogen samples containing the rinsed coated catheter components. As shown, the antiseptic coating material of formulation 9 demonstrated significant inhibition of the *S. aureus* and *E. coli* pathogens over the seven day period, despite a prewashing of the catheter components. Again, the *P. aeruginosa* and C. *albicans* pathogens demonstrated little or no inhibition to the coating material following the prewashing. These results confirm both the cohesive nature of the coating material due to the silicone oil component, and the mutual miscibility of the antipathogenic agent with the remaining components of the coating material.

Finally, Table 4 shows the results from the pathogen samples containing the uncoated catheter components, or the control sample. As shown, without the coating material of formulation 9, all samples demonstrated no inhibition. Accordingly, the antiseptic coating material of the present invention has proven to be highly effective at killing or preventing the proliferation of several pathogens that commonly cause bloodstream infections.

## Claims

1. An antimicrobial composition comprising:
an antipathogenic agent;
a first solvent capable of dissolving the antipathogenic agent, the solvent being selected from a group consisting of water, a C1 alcohol and a C2 alcohol;
a lubricious agent represented by a silicone oil; and
a second solvent capable of dissolving the lubricious agent, wherein the first solvent and the second solvent are mutually miscible.

2. The composition of claim 1, wherein the second solvent is at least one of a hydrocarbon, a ketone, a halogenated hydrocarbon, and an alcohol having at least 3 carbon atoms.

3. The composition of claim 1, wherein the second solvent is a polyethoxylated surfactant.

4. The composition of claim 1, wherein the antipathogenic agent is at least one of chlorhexidine diacetate, chlorhexidine gluconate, triclosan, benzalkonium chloride, and para-chloro-meta-xylenol (PCMX).

5. The composition of claim 1, wherein the silicone oil is at least one of dimethylsiloxane and trifluoropropylmethylsiloxane.

6. The composition of claim 2, wherein the second solvent comprises two or more hydrocarbons, ketones, halogenated hydrocarbons, alcohols having at least three carbon atoms, and combinations thereof.

7. A method for manufacturing the antimicrobial composition as defined in any one of claims 1 to 6, the method comprising:
selecting the antipathogenic agent;
selecting the first solvent capable of dissolving the antipathogenic agent, the solvent being selected from a group consisting of water, a C1 alcohol and a C2 alcohol;
dissolving the antipathogenic agent in the first solvent to provide a first miscible solution;
selecting the lubricious agent represented by a silicone oil;
selecting the second solvent capable of dissolving the lubricious agent;
dissolving the lubricious agent in the second solvent to provide a second miscible solution; and
homogeneously mixing the first and second miscible solutions.

8. An intravascular system for preventing catheter-related bloodstream infections, the system comprising:
a vascular access device having an external surface; and
an antiseptic coating applied to the external surface, the antiseptic coating including;
an antipathogenic agent;
a first solvent capable of dissolving the antipathogenic agent, the solvent being selected from a group consisting of water, a C1 alcohol and a C2 alcohol;
a lubricious agent represented by a silicone oil; and
a second solvent capable of dissolving the lubricious agent, wherein the first solvent and the second solvent are mutually miscible.

9. The system of claim 8, wherein the vascular access device further comprises a syringe.

10. The system of claim 8, wherein the second solvent is at least one of a hydrocarbon, a ketone, a halogenated hydrocarbon, and an alcohol having at least 3 carbon atoms.

11. The system of claim 8, wherein the second solvent is a polyethoxylated surfactant.

12. The system of claim 8, wherein the silicone oil is at least one of dimethylsiloxane and trifluoropropylmethylsiloxane.

13. Use of the composition of any one of claims 1 to 6 for applying an antiseptic lubricious coating on surfaces.

## Patentansprüche

1. Antimikrobielle Zusammensetzung, umfassend:
ein antipathogenes Mittel;
ein erstes Lösungsmittel, das das antipathogene Mittel auflösen kann, wobei das Lösungsmittel aus einer Gruppe ausgewählt ist, die aus Wasser, einem C1-Alkohol und einem C2-Alkohol besteht;
ein Schmiermittel, das von einem Silikonöl dargestellt wird; und
ein zweites Lösungsmittel, das das Schmiermittel auflösen kann, wobei das erste Lösungsmittel und das zweite Lösungsmittel miteinander mischbar sind.

2. Zusammensetzung gemäß Anspruch 1, wobei das zweite Lösungsmittel wenigstens eines aus einem Kohlenwasserstoff, einem Keton, einem halogenierten Kohlenwasserstoff und einem Alkohol mit wenigstens 3 Kohlenstoffatomen ist.

3. Zusammensetzung gemäß Anspruch 1, wobei das zweite Lösungsmittel ein polyethoxyliertes Tensid ist.

4. Zusammensetzung gemäß Anspruch 1, wobei das antipathogene Mittel wenigstens eines aus Chlorhexidindiacetat, Chlorhexidingluconat, Triclosan, Benzalkoniumchlorid und para-Chlor-meta-xylenol (PCMX) ist.

5. Zusammensetzung gemäß Anspruch 1, wobei das Silikonöl wenigstens eines aus Dimethylsiloxan und Trifluorpropylmethylsiloxan ist.

6. Zusammensetzung gemäß Anspruch 2, wobei das zweite Lösungsmittel zwei oder mehr Kohlenwasserstoffe, Ketone, halogenierte Kohlenwasserstoffe, Alkohole mit wenigstens drei Kohlenstoffatomen und Kombinationen davon umfasst.

7. Verfahren zur Herstellung der antimikrobiellen Zusammensetzung gemäß einem der Ansprüche 1 bis 6, wobei das Verfahren umfasst:
Auswählen des antipathogenen Mittels;
Auswählen des ersten Lösungsmittels, das das antipathogene Mittel auflösen kann, wobei das Lösungsmittel aus einer Gruppe ausgewählt ist, die aus Wasser, einem C1-Alkohol und einem C2-Alkohol besteht;
Auflösen des antipathogenen Mittels im ersten Lösungsmittel, was eine erste mischbare Lösung ergibt;
Auswählen des Schmiermittels, das von einem Silikonöl dargestellt wird;
Auswählen des zweiten Lösungsmittels, das das Schmiermittel auflösen kann;
Auflösen des Schmiermittels im zweiten Lösungsmittel, was eine zweite mischbare Lösung ergibt; und
homogenes Mischen der ersten und der zweiten mischbaren Lösung.

8. Intravaskuläres System zur Prävention von mit Kathetern zusammenhängenden Blutstrominfektionen, wobei das System umfasst:
eine Gefäßzugangsvorrichtung mit einer externen Fläche; und
eine antiseptische Beschichtung, die auf die externe Fläche aufgetragen ist, wobei die antiseptische Beschichtung umfasst:
ein antipathogenes Mittel;
ein erstes Lösungsmittel, das das antipathogene Mittel auflösen kann, wobei das Lösungsmittel aus einer Gruppe ausgewählt ist, die aus Wasser, einem C1-Alkohol und einem C2-Alkohol besteht;
ein Schmiermittel, das von einem Silikonöl dargestellt wird; und
ein zweites Lösungsmittel, das das Schmiermittel auflösen kann, wobei das erste Lösungsmittel und das zweite Lösungsmittel miteinander mischbar sind.

9. System gemäß Anspruch 8, wobei die Gefäßzugangsvorrichtung weiterhin eine Spritze umfasst.

10. System gemäß Anspruch 8, wobei das zweite Lösungsmittel wenigstens eines aus einem Kohlenwasserstoff, einem Keton, einem halogenierten Kohlenwasserstoff und einem Alkohol mit wenigstens 3 Kohlenstoffatomen ist.

11. System gemäß Anspruch 8, wobei das zweite Lösungsmittel ein polyethoxyliertes Tensid ist.

12. System gemäß Anspruch 8, wobei das Silikonöl wenigstens eines aus Dimethylsiloxan und Trifluorpropylmethylsiloxan ist.

13. Verwendung der Zusammensetzung gemäß einem der Ansprüche 1 bis 6 zum Auftragen einer antiseptischen schmierenden Beschichtung auf Oberflächen.

## Revendications

1. Composition antimicrobienne comprenant :
un agent antipathogène ;
un premier solvant capable de dissoudre l'agent antipathogène, le solvant étant choisi dans un groupe constitué d'eau, d'un alcool en C1 et d'un alcool en C2 ;
un agent lubrifiant représenté par une huile de silicone ; et
un deuxième solvant capable de dissoudre l'agent lubrifiant, où le premier solvant et le deuxième solvant sont mutuellement miscibles.

2. Composition de la revendication 1, dans laquelle le deuxième solvant est au moins l'un(e) d'un hydrocarbure, d'une cétone, d'un hydrocarbure halogéné et d'un alcool ayant au moins 3 atomes de carbone.

3. Composition de la revendication 1, dans laquelle le deuxième solvant est un tensio-actif polyéthoxylé.

4. Composition de la revendication 1, dans laquelle l'agent antipathogène est au moins l'un du diacétate de chlorhexidine, du gluconate de chlorhexidine, du triclosan, du chlorure de benzalkonium, et du parachlorométaxylénol (PCMX).

5. Composition de la revendication 1, dans laquelle l'huile de silicone est au moins l'un du diméthylsiloxane et du trifluoropropylméthylsiloxane.

6. Composition de la revendication 2, dans laquelle le deuxième solvant comprend deux éléments ou plus parmi des hydrocarbures, des cétones, des hydrocarbures halogénés, des alcools ayant au moins trois atomes de carbone, et des combinaisons de ceux-ci.

7. Procédé de fabrication de la composition antimicrobienne telle que définie dans l'une quelconque des revendications 1 à 6, le procédé comprenant le fait :
de sélectionner l'agent antipathogène ;
de sélectionner le premier solvant capable de dissoudre l'agent antipathogène, le solvant étant choisi dans un groupe constitué d'eau, d'un alcool en C1 et d'un alcool en C2 ;
de dissoudre l'agent antipathogène dans le premier solvant pour fournir une première solution miscible ;
de sélectionner l'agent lubrifiant représenté par une huile de silicone ;
de sélectionner le deuxième solvant capable de dissoudre l'agent lubrifiant ;
de dissoudre l'agent lubrifiant dans le deuxième solvant pour fournir une deuxième solution miscible ; et
de mélanger de façon homogène les première et deuxième solutions miscibles.

8. Système intravasculaire pour la prévention des infections sanguines liées au cathéter, le système comprenant :
un dispositif d'accès vasculaire ayant une surface externe ; et
un revêtement antiseptique appliqué sur la surface externe, le revêtement antiseptique comportant ;
un agent antipathogène ;
un premier solvant capable de dissoudre l'agent antipathogène, le solvant étant choisi dans un groupe constitué d'eau, d'un alcool en C1 et d'un alcool en C2 ;
un agent lubrifiant représenté par une huile de silicone ; et
un deuxième solvant capable de dissoudre l'agent lubrifiant, où le premier solvant et le deuxième solvant sont mutuellement miscibles.

9. Système de la revendication 8, dans lequel le dispositif d'accès vasculaire comprend en outre une seringue.

10. Système de la revendication 8, dans lequel le deuxième solvant est au moins l'un(e) d'un hydrocarbure, d'une cétone, d'un hydrocarbure halogéné et d'un alcool ayant au moins 3 atomes de carbone.

11. Système de la revendication 8, dans lequel le deuxième solvant est un tensio-actif polyéthoxylé.

12. Système de la revendication 8, dans lequel l'huile de silicone est au moins l'un du diméthylsiloxane et du trifluoropropylméthylsiloxane.

13. Utilisation de la composition de l'une quelconque des revendications 1 à 6 pour appliquer un revêtement lubrifiant antiseptique sur des surfaces.
